# EUROPEAN PATENT SPECIFICATION

(11) **EP 2 140 823 B1**
(45) Date of publication and mention of the grant of the patent: **26.08.2015**
(21) Application number: 08161858.9
(22) Date of filing: 05.08.2008
(51) Int. Cl.: A61B 17/34, A61B 17/56

(54) **Pin assembly for operation**
Stiftanordnung für Operationen
Ensemble de broche pour opérations

(30) Priority: 23.06.2008 KR 20080059222
(43) Date of publication of application: 06.01.2010
(73) Proprietor: National Cancer Center, Goyang-si, Gyeonggi-do 410-352 (KR)
(72) Inventor: Kang, Hyun Guy 960-3, Madu 1-dong, 410-351, Goyang-si (KR)
(74) Representative: Bassil, Nicholas Charles

(56) References cited:
- WO-A-00/54705
- WO-A-2005/053545
- WO-A-2006/011152
- WO-A-2007/122608
- FR-A- 2 820 630
- US-A1- 2004 002 713

## Description

### BACKGROUND OF THE INVENTION

### Field of the Invention

The present invention relates to a pin assembly for operation and, more particularly, to a bendable pin assembly for operation in which a plurality of holes are defined through the wall and the root part thereof so that various medication and treatment agent can be easily injected.

The closest prior art is document WO 2006/011152 A2, which defines the preamble of independent claims 1 and 8.

### Description of the Related Art

These days, despite the development of diagnosis and treatment techniques for cancer patients whose number gradually increases, the number of patients who suffer pain due to spreading of cancer to the bone also increases. In America, 1.2 millions of cancer patients occur every year, and among these people, bone metastasis has been reported in six hundred thousands constituting 50%.

If cancer cells spread to the bone, the bone is likely to be adsorbed and weakened to develop to fracture or imminent fracture. In this case, though surgical treatment is necessary, since the general condition of a patient is unstable, the patient cannot be ready to receive a major operation. Otherwise, even when the patient is ready to receive the major operation, because extensive incision of the skin and the muscle is needed, anticancer medicine treatment which is essential to the treatment of metastasis cannot be conducted for a certain period before and after the operation. Due to this fact, a number of patients suffering bone metastasis cannot properly lead basic everyday life while not receiving surgical treatment and must be ready to suffer serious pains.

In addition to the bone metastasis, as the aging of population proceeds, the number of patients suffering senile fracture and osteoporotic fracture increases. In this case, aged patients have a bone union period longer than that of young people, and even when surgical treatment is conducted, it is the norm to securely hold a fractured portion for an extended period. Due to this fact, in the course of treating the patients suffering senile fracture or osteoporotic fracture, complications such as fracture nonunion, unstable general condition and weakened limb activity may be caused.

Therefore, endeavors for increasing the satisfactoriness of patients by quickly and simply conducting surgical treatment for the fractured or damaged bone without causing complications while preserving stable general condition of aged patients or cancer patients and not hindering anticancer medicine treatment are keenly demanded.

However, in methods for reinforcing a bone weakened in the course of eliminating cancer cells spread to the bone or treating a fracture and for operating a fractured bone, which have disclosed so far in the art, after the skin and the muscle are incised over an extensive range, tumor is curetted, or a fractured portion of the bone is securely held using an artificial joint such as a large metal implant or plate, a tumor replacement, and the like, or is supported using an artificial aid.

In these methods, research has been conducted such that material for promoting bone regeneration or agent for suppressing bone resorption is directly coated at an area where the artificial joint is driven into the bone. Alternatively, after a metal pin is inserted into the fractured portion, the bone replacement as a bone regeneration inducer is injected separately by defining a hole in the bone. Also, in the case of osteoporotic vertebral compression fracture or spine metastasis, a method has been used, in which only bone cement is injected into the vertebral body through a pedicle in an effort to reinforce the spine.

Therefore, due to the extensive operation, in order to receive anticancer medicine treatment after the operation is conducted, it is necessary to wait until the wound is completely recovered. Also, because of side effects due to anesthesia and bleeding, the general condition of a patient is likely to be rapidly worsened so that the patient's life can be placed under a dangerous situation. Furthermore, when securing the artificial aid to the fractured portion of the bone, if the patient suffers serious osteoporosis, since the bone is weak, fasteners are apt to be released.

### SUMMARY OF THE INVENTION

Accordingly, the present invention has been made keeping in mind the above problems occurring in the related art, and an object of the present invention is to provide a pin assembly for operation which can solve the problems caused due to extensive surgery and allow medication or treatment agent to be easily injected to a treatment position, wherein, after a pin for operation is percutaneously inserted without the need of incision of the skin or the muscle, various medication is injected into the bone through the fastened pin to cause the death of cancer cells, and by injecting bone cement or anticancer medicine mixed with bone cement, a fractured bone can be securely held and the strength of the fractured bone can be increased.

Another object of the present invention is to provide a pin assembly for operation which allows medication such as bone regenerating material and anti-resorptive agent to be easily injected without the need of separately defining an opening in a fractured portion after the fractured portion is securely held in the case of senile fracture caused due to osteoporosis.

In order to achieve the above objects, according to one aspect of the present invention, there is provided a pin assembly for operation as defined in claim 1.

According to another aspect of the present invention, the root part of the pin for operation is sharply or bluntly formed, and a thread is formed on the root part of the pin for operation.

According to another aspect of the present invention, the pin for operation is made of stainless steel, titanium, or a titanium-based alloy.

According to another aspect of the present invention, medication or bone cement is injected through the holes defined in the pin for operation into the bone.

According to another aspect of the present invention, the medication is selected from the group consisting of alcohol, liquid nitrogen, anticancer medicine, bone regenerating material, and anti-resorptive agent.

In order to achieve the above objects, according to another aspect of the present invention, there is provided a pin assembly for operation as defined in claim 8.

According to another aspect of the present invention, the pin for operation is made of stainless steel, titanium, or a titanium-based alloy.

According to still another aspect of the present invention, medication or bone cement is injected through the holes defined in the pin for operation into the bone.

According to still a further aspect of the present invention, the medication is selected from the group consisting of alcohol, liquid nitrogen, anticancer medicine, bone regenerating material, and anti-resorptive agent.

### BRIEF DESCRIPTION OF THE DRAWINGS

The above and other objects, features and other advantages of the present invention will be more clearly understood from the following detailed description taken in conjunction with the accompanying drawings, in which:
FIGs. 1a through 1f are schematic perspective views illustrating various configurations of a pin which is used in a pin assembly for operation;
FIG. 2 is a sectional view illustrating a pin assembly for operation;
FIG. 3 is a sectional view illustrating a pin assembly for operation in accordance with the present invention;
FIG. 4 is a perspective view illustrating a driver to be used in a pin assembly for operation according to the present invention;
FIG. 5 is a perspective view illustrating a pin assembly for operation, with a pin coupled to the driver shown in FIG. 4; and
FIG. 6 is a radiograph obtained by penetrating X-rays through a bone having pins driven therein.

### DETAILED DESCRIPTION OF PREFERRED EMBODIMENTS

Reference will now be made in greater detail to preferred embodiments of the invention, examples of which are illustrated in the accompanying drawings. Wherever possible, the same reference numerals will be used throughout the drawings and the description to refer to the same or like parts.

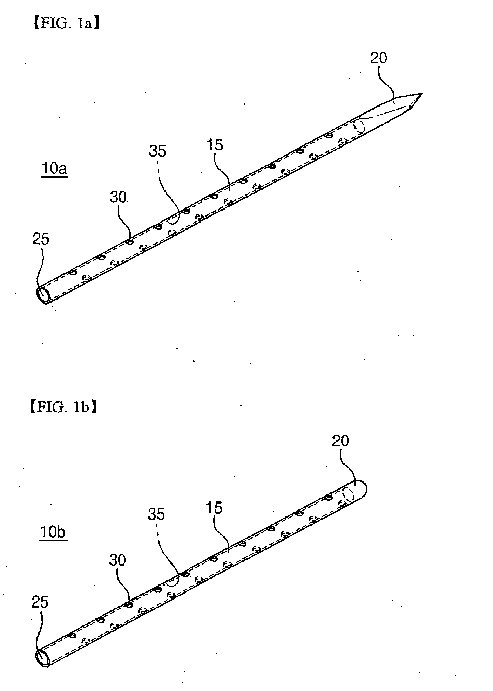

FIGs. 1a through 1f are schematic perspective views illustrating various configurations of a pin which is used in a pin assembly for operation according to the present invention.

Referring to FIGs. 1a through 1f, each of pins 10 for operation has a configuration of a hollow elongate bar which has a plurality of side holes 30 defined through the wall thereof. Each pin 10 for operation has a straight body part 15 which has the side holes 30 defined through the wall thereof and a root part 20 which is formed on the distal end of the body part 15 and is brought into contact with the bone or another portion of the human body.

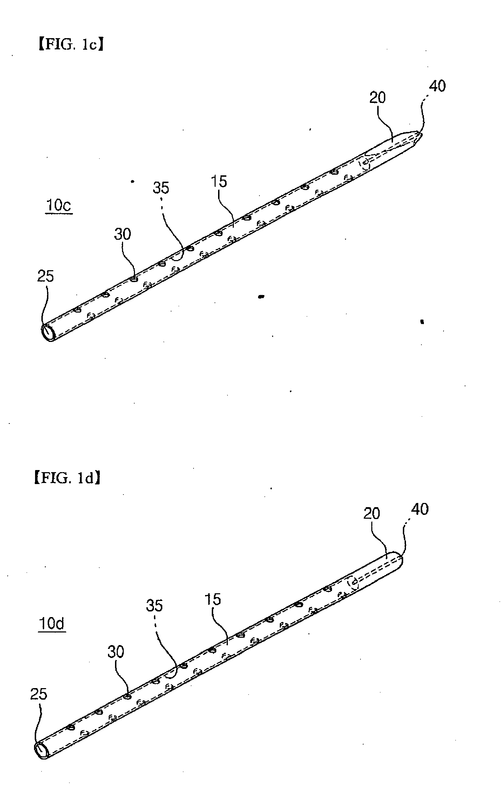

The pins 10 for operation shown in FIGs. 1a through 1f are distinguished from one another by the shapes of respective root parts 20. FIG. 1a illustrates a pin 10a for operation, in which the root part 20 is sharply formed and the distal end of the root part 20 is closed. FIG. 1b illustrates a pin 10b for operation, in which the root part 20 is bluntly formed and the distal end of the root part 20 is closed. FIG. 1c illustrates a pin 10c for operation, in which the root part 20 is sharply formed and a through-hole 40 is defined through the root part 20 so that the distal end of the root part 20 is open. FIG. 1d illustrates a pin 10d for operation, in which the root part 20 is bluntly formed and a through-hole 40 is defined through the root part 20 so that the distal end of the root part 20 is open.

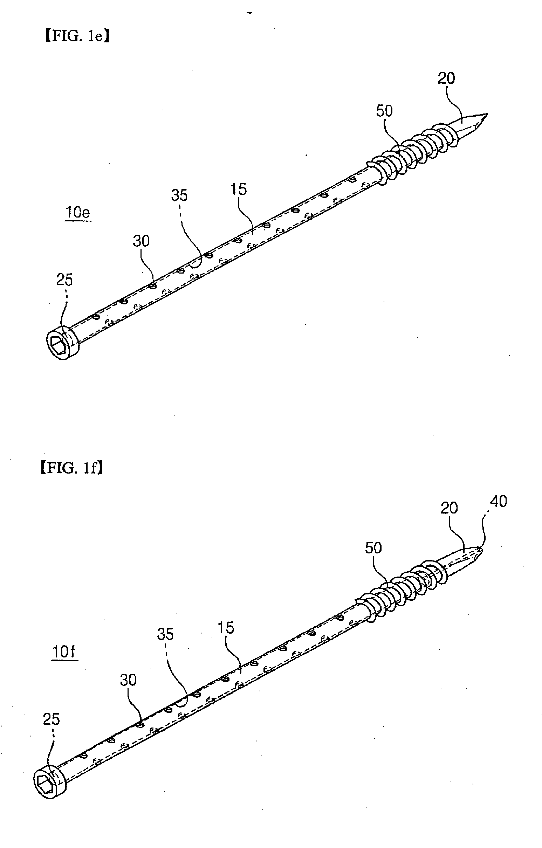

FIG. 1e illustrates a pin 10e for operation, in which the root part 20 is sharply formed, a thread 50 is formed on the root part 20, and the distal end of the root part 20 is closed. FIG. 1f illustrates a pin 10f for operation, in which the root part 20 is sharply formed, a thread 50 is formed on the root part 20, and a through-hole 40 is defined through the root part 20 so that the distal end of the root part 20 is open.

As a surgeon inserts the pin 10 for operation shown in each of FIGs. 1a through 1f into the bone of a patient and injects medication or bone cement through an inlet port 25 which is defined at the proximal end of the pin 10 for operation, the medication or bone cement flows through an internal passage 35 defined in the pin 10 for operation and is discharged into the bone through the side holes 30 and the through-hole 40.

The injection amount of the medication or bone cement or a treatment position can be adjusted by changing the sizes and the positions of the side holes 30 and the through-hole 40 so that medial treatment can be given in accordance with an order of priority.

The pin 10 for operation can be made of stainless steel and, as a matter of course, may be made of an antirust alloy. For instance, titanium or a titanium-based alloy can be used.

The pin 10 for operation can be bent, specifically, not to project out of a curved bone. In this regard, the pin 10 for operation can be bent in an appropriate range using a press bender or another tool prepared in an operation room.

A guide pin 70 or a reinforcing metal wire is additionally provided in a manner such that it is inserted through the through-hole 40 of the pin 10 for operation to reinforce the strength of the pin 10 for operation. In the same manner as the pin 10 for operation, the guide pin 70 or the reinforcing metal wire can be made of stainless steel or an antirust alloy. It is preferred that titanium or a titanium-based alloy be used.

In the case that the guide pin 70 or the reinforcing metal wire is inserted through the internal passage 35 which is defined in the pin 10 for operation, the guide pin 70 or the reinforcing metal wire can be bent simultaneously with the pin 10 for operation. In this case, the guide pin 70 or the reinforcing metal wire can be bent using a press bender in an allowable range in conformity with a degree to which a bone as the target of operation is curved.

Bone cement can be introduced into the bone through the internal passage 35 which is defined in the pin 10 for operation. The bone cement reinforces the bone, prevents the inserted pin 10 for operation from being released out of the bone, increases the coupling strength between the bone and the pin 10 for operation, and generates heat while being set, so as to cause the death of cancer cells.

The medication capable of being injected through the pin 10 for operation includes 100% alcohol which can cause the death of cancer cells, liquid nitrogen which can quickly freezing cancer cells and cause the death of the cancer cells, anticancer medicine which can be introduced into the bone while being mixed with bone cement, to continuously cause the death of cancer cells, bone regenerating material which can promote bone union in a fractured portion of the bone, and anti-resorptive agent which can prevent osteoporosis and bone destruction from proceeding.

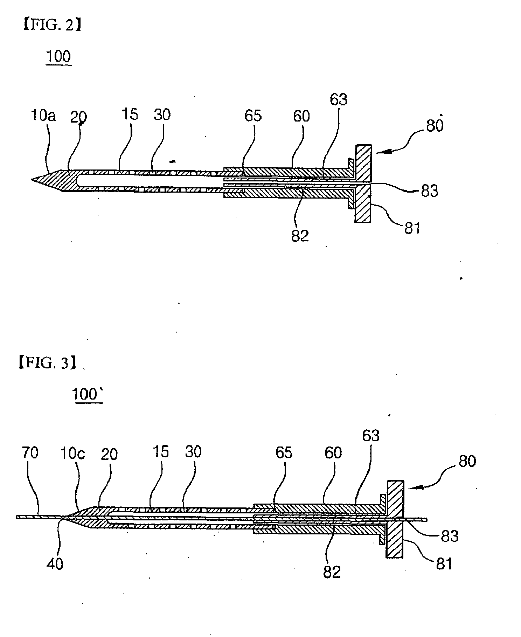

FIG. 2 is a sectional view illustrating a pin assembly 100 for operation not in accordance with the present invention, and FIG. 3 is a sectional view illustrating a pin assembly 100' for operation in accordance with the present invention.

The pin assembly 100 for operation shown in FIG. 2 is composed of a hollow pin 10a for operation which has a plurality of side holes 30 defined through the wall thereof and in which a root part 20 is sharply formed and is closed, a hollow support member 60 in which a pin fastening part 65 is formed in the shape of a groove to fasten the proximal end of the pin 10a for operation and both ends of the support member 60 are open, and a T-shaped impactor 80 which is inserted into an insertion hole 63 defined through the support member 60 and has a through-hole 83 defined through the center portion thereof.

In the case that the impactor 80 is not provided, the pin 10a for operation is inserted into the bone by applying external force to the proximal end of the support member 60. In the case that the impactor 80 is provided, the pin 10a for operation is inserted into the bone by applying external force to a head part 81 of the impactor 80.

It is preferred that the support member 60 and the impactor 80 be made of material which is not easily deformed or broken by the application of external force.

After the pin 10a for operation is driven into the bone to be treated, the impactor 80 is removed, and medication or bone cement is injected into the pin 10a for operation through the support member 60 which is hollow to be open at both ends thereof. The injected medication or bone cement flows through the side holes 30 to a treatment position in the bone so as to cause the death of cancer cells or be set in the bone.

As the pin for operation used in the pin assembly for operation shown in FIG. 2, all the pins 10a through 10f for operation shown in FIGs. 1A through 1g can be adopted.

The pin assembly 100' for operation shown in FIG. 3 is composed of a hollow pin 10c for operation which has a plurality of side holes 30 defined through the wall thereof and in which a root part 20 is sharply formed and a through-hole 40 is defined through the root part 20, a hollow support member 60 in which a pin fastening part 65 is formed in the shape of a groove to fasten the proximal end of the pin 10c for operation and both ends of the support member 60 are open, and a T-shaped impactor 80 which is inserted into an insertion hole 63 defined through the support member 60 and has a through-hole 83 defined through the center portion thereof.

The pin assembly 100' for operation includes a guide pin 70 or a reinforcing metal wire which is installed by being inserted through the through-hole 83 of the impactor 80 and the through-hole 40 defined through the root part 20 of the pin 10c for operation. After the surgeon drives the guide pin 70 through the skin into the bone, by applying external force to the support member 60 or the impactor 80, the pin 10c for operation is driven into the bone along the path of the guide pin 70.

The guide pin 70 functions to guide the pin 10c for operation so that the pin 10c for operation can be easily driven into the bone. In this regard, as the occasion demands, by not removing the guide pin 70 but leaving an appropriate length of the guide pin 70, the guide pin 70 can serve to reinforce the strength of the pin 10c for operation in the same way as the reinforcing metal wire.

When it is necessary to treat a curved bone, it is preferable to use a curved pin rather than a straight pin since medication or bone cement can be uniformly distributed and treatment efficiency can be improved.

Hence, after inserting the guide pin 70 into the pin 10c for operation, the guide pin 70 and the pin 10c for operation are simultaneously bent using a press bender, etc., and then, by applying external force to them, they can be driven into the bone.

After the pin 10c for operation is driven into the bone to be treated, medication or bone cement is injected through the support member 60. The injected medication or bone cement flows through the inlet port 25, the side holes 30 and the through-hole 40 defined through the root part 20 to a treatment position in the bone so as to cause the death of cancer cells or be set in the bone.

The pins 10 for operation, which are used in the pin assemblies 100 and 100' for operation, can be replaced with other ones depending upon the uses thereof. For example, in the case that a thread 50 is formed on the root part 20 of the pin 10 for operation, the pin 10 for operation can be threadedly driven into the bone. This pin 10 for operation can be adopted when the bone is seriously weakened.

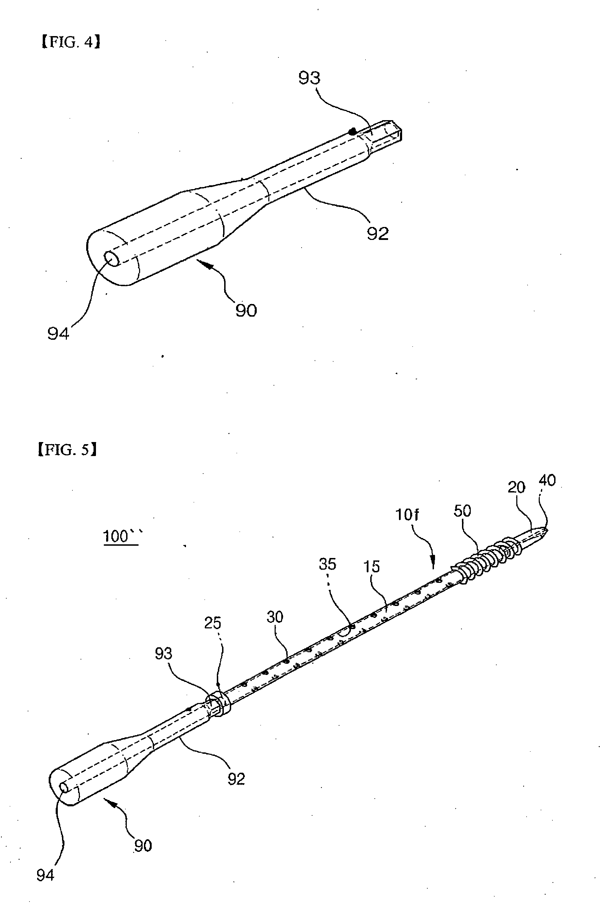

FIG. 4 is a perspective view illustrating a driver to be used in a pin assembly for operation according to the present invention, and FIG. 5 is a perspective view illustrating a pin assembly for operation, with a pin coupled to the driver shown in FIG. 4.

Referring to FIGs. 4 and 5, a pin assembly 100" for operation includes a hollow pin 10f for operation which has a plurality of side holes 30 defined through the wall thereof and in which a thread 50 is formed on a root part 20, and a hollow driver 90 which is fitted into the proximal end of the pin 10f for operation to transmit external force to the pin 10f for operation and has a through-hole 94 defined therethrough.

A through-hole 40 is defined through the root part 20 of the pin 10f for operation, and an inlet port 25 of the pin 10f for operation has a hexagonal opening.

The driver 90 has a cylindrical body part 92 which has different diameters at both ends thereof. A through-hole 94 is defined through the center portion of the driver 90. An insertion part 93 having a hexagonal sectional shape is formed at the distal end of the body part 92.

In the state in which the insertion part 93 of the driver 90 is fitted into the inlet port 25 of the pin 10f for operation and the root part 20 of the pin 10f for operation is brought into direct contact with a portion of the bone to be treated, by applying external force to the driver 90, the pin 10f for operation can be driven into the bone. At this time, since force is transmitted through the driver 90 comprising a unitary element, the force can be concentrated rather than dispersed. Therefore, even though the thread 50 is formed on the root part 20 of the pin 10f for operation, the pin 10f for operation can be easily driven into the bone.

The driver 90 is mainly used for a pin for operation which is formed with the thread 50. Thus, the pin 10e for operation shown in FIG. 1E can also be used together with the driver 50.

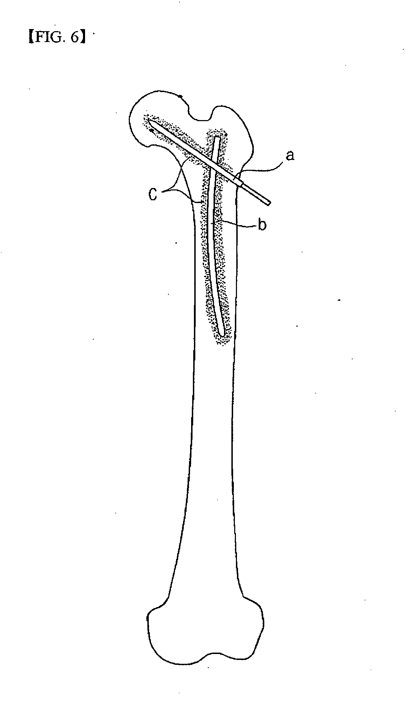

FIG. 6 is a radiograph obtained by penetrating X-rays through a bone having pins 10 driven therein. Referring to FIG. 6, a curved pin 'b' for operation is completely inserted into the bone, and a straight pin 'a' for operation is partially inserted into the bone.

Regions 'C' around the pins 'a' and 'b' for operation indicate the distribution of the medication or bone cement which is injected into the bone through the side holes defined in the pins 'a' and 'b' for operation.

The pin for operation and the pin assembly for operation according to the present invention can be used only by defining a hole in the skin without the need of extensive incision of the skin or the muscle. Further, by injecting various medication or treatment agent in various ways through the internal passage and the side holes defined in the pin for operation, the pin for operation and the pin assembly for operation according to the present invention can be effectively used for bone metastasis, senile fracture, osteoporotic fracture, fracture nonunion, etc. As a consequence, since operation can be conveniently conducted within a short time without bleeding, advantages are conferred in that the general condition of a patient can be preserved and, when subsequent treatment is necessary, it can be immediately taken without loss of time.

As is apparent from the above description, the pin assembly for operation according to the present invention can be used only by defining a hole in the skin without the need of extensive incision of the skin or the muscle. By injecting or introducing medication for causing the death of cancer cells in a variety of ways through an internal passage and side holes defined in a pin for operation, the cancer cells can be effectively eliminated. Also, by injecting bone cement, etc., a portion of the bone that is weakened by cancer or is fractured by an accident can be reinforced or securely held. Moreover, in the case of senile fracture and osteoporotic fracture, after operation for securely holding a fractured portion of the bone is conducted, it is not necessary to separately define an opening in the weakened bone, and instead, by inserting the pin for operation into the bone, the fractured portion can be securely held and bone regenerating material or anti-resorptive agent can be injected into the bone.

Although preferred embodiments of the present invention have been described for illustrative purposes, those skilled in the art will appreciate that various modifications, additions and substitutions are possible, without departing from the scope of the invention as disclosed in the accompanying claims.

## Claims

1. A pin assembly for operation, comprising:
a hollow pin (10) for operation having a plurality of side holes (30) defined through a wall thereof and a root part (20) which is open due to the presence of a through-hole (40);
a hollow support member (60) having a pin fastening part (65) formed therein in the shape of a groove to fasten one end of the pin for operation, and being open at both ends thereof;
a T-shaped impactor (80) adapted to be inserted into an insertion hole (63) defined through the support member, wherein the pin for operation is adapted to be fastened to the pin fastening part of the support member, and by applying external force to the impactor, the pin for operation is adapted to be inserted into a bone; **characterised in that** the T-shaped inpactor has a through-hole (83) defined through a center portion thereof; and the pin assembly further comprises
a guide pin (70) or a reinforcing metal wire adapted to be inserted through the through-hole defined through the root part of the pin for operation,
wherein:
the guide pin or reinforcing metal wire is adapted to reinforce the strength of the pin for operation when in situ; and
the pin for operation, the guide pin and the reinforcing metal wire are adapted to bent.

2. The pin assembly according to claim 1, wherein the pin assembly is adapted such that medication or bone cement may remain in the hollow pin for operation after the pin for operation is inserted into the bone.

3. The pin assembly according to claim 1 or 2, wherein the root part of the pin for operation is sharply or bluntly formed.

4. The pin assembly according to claim 1 or 2, wherein a thread (50) is formed on the root part of the pin for operation.

5. The pin assembly according to claim 1 or 2, wherein the pin for operation is made of stainless steel, titanium, or a titanium-based alloy.

6. The pin assembly according to claim 1 or 2, wherein medication or bone cement may be injected through the holes defined in the pin for operation into the bone.

7. The pin assembly according to claim 6, wherein the medication is selected from the group consisting of alcohol, liquid nitrogen, anticancer medicine, bone regenerating material, and anti-resorptive agent.

8. A pin assembly for operation, comprising:
a hollow pin (10) for operation having a plurality of side holes (30) defined through a wall thereof and a root part (20) which is open due to presence of a through-hole (40); **characterised in that** a thread is formed on the root part and the pin assembly for operation further comprises
a driver (90) adapted to be fitted into one open end of the pin for operation to transmit external force to the pin for operation, the driver having a through-hole (94); and
a guide pin (70) or a reinforcing metal wire adapted to be inserted through the through-hole defined through the root part of the pin for operation;
wherein:
by applying external force to the driver, the pin for operation is adapted to be driven into a bone to hold a fractured portion; and
the pin for operation, the guide pin and the reinforcing metal wire are adapted to be bent.

9. The pin assembly according to claim 8, wherein the pin for operation is made of stainless steel, titanium, or a titanium-based alloy.

10. The pin assembly according to claim 8, wherein the pin assembly is adapted such that medication or bone cement may remain in the hollow pin for operation after the pin for operation is inserted into the bone.

11. The pin assembly according to claim 8, wherein medication or bone cement may be injected through the holes defined in the pin for operation into the bone.

12. The pin assembly according to claim 11, wherein the medication is selected from the group consisting of alcohol, liquid nitrogen, anticancer medicine, bone regenerating material, and anti-resorptive agent.

## Patentansprüche

1. Chirurgische Stiftanordnung, umfassend:
einen chirurgischen Hohlstift (10) mit einer Vielzahl von Seitenlöchern (30), die durch seine Wand definiert sind, und einem Wurzelteil (20), der aufgrund des Vorhandenseins einer Durchgangsbohrung (40) offen ist;
ein hohles Stützelement (60) mit einem darin in Form einer Nut ausgebildeten Stiftbefestigungsteil (65), um ein Ende des chirurgischen Stifts zu befestigen, und das an seinen beiden Enden offen ist;
einen T-förmigen Impaktor (80), der geeignet ist zum Einsetzen in ein durch das Stützelement definiertes Einsteckloch (63), wobei der chirurgische Stift dafür angepasst ist, an dem Stiftbefestigungsteil des Stützelements befestigt zu werden, und durch Anwenden von äußerer Kraft an dem Impaktor der chirurgische Stift dafür geeignet ist, in einen Knochen inseriert zu werden, **dadurch gekennzeichnet, dass** der T-förmige Impaktor eine durch seinen zentralen Bereich definierte Durchgangsbohrung (83) aufweist; und die Stiftanordnung außerdem umfasst:
einen Führungsstift (70) oder einen verstärkenden Metalldraht, der dafür angepasst ist, durch die durch den Wurzelteil des chirurgischen Stifts definierte Durchgangsbohrung inseriert zu werden,
wobei:
der Führungsstift oder der verstärkende Metalldraht dafür geeignet ist, die Festigkeit des chirurgischen Stifts zu verstärken, wenn in situ; und
der chirurgische Stift, der Führungsstift und der verstärkende Metalldraht dafür geeignet sind, gekrümmt zu werden.

2. Stiftanordnung gemäß Anspruch 1, wobei die Stiftanordnung so angepasst ist, dass eine Medikation oder ein Knochenzement in dem chirurgischen Hohlstift verbleiben kann, nachdem der chirurgische Stift in den Knochen inseriert ist.

3. Stiftanordnung gemäß Anspruch 1 oder 2, worin der Wurzelteil des chirurgischen Stifts spitz oder stumpf geformt ist.

4. Stiftanordnung gemäß Anspruch 1 oder 2, wobei ein Gewinde (50) an dem Wurzelteil des chirurgischen Stifts ausgebildet ist.

5. Stiftanordnung gemäß Anspruch 1 oder 2, wobei der chirurgische Stift aus Edelstahl, Titan oder einer Legierung auf Titanbasis hergestellt ist.

6. Stiftanordnung gemäß Anspruch 1 oder 2, wobei die Medikation oder der Knochenzement durch die in dem chirurgischen Stift definierten Löcher in den Knochen injiziert werden kann.

7. Stiftanordnung gemäß Anspruch 6, wobei die Medikation ausgewählt ist aus der Gruppe, bestehend aus Alkohol, Flüssigstickstoff, Antikrebs-Medikament, knochenregenerierendem Material und antiresorptivem Mittel.

8. Chirurgische Stiftanordnung, umfassend:
einen chirurgischen Hohlstift (10) mit einer Vielzahl von Seitenlöchern (30), die durch seine Wand definiert sind, und einem Wurzelteil (20), der aufgrund des Vorhandenseins einer Durchgangsbohrung (40) offen ist; **dadurch gekennzeichnet, dass** ein Gewinde an dem Wurzelteil ausgebildet ist und die chirurgische Stiftanordnung außerdem umfasst:
einen Steckgriff (90), der dafür geeignet ist, in ein offenes Ende des chirurgischen Stifts eingepasst zu werden, um äußere Kraft auf den chirurgischen Stift zu übertragen, wobei der Steckgriff eine Durchgangsbohrung (94) aufweist; und
einen Führungsstift (70) oder einen verstärkenden Metalldraht, der dafür angepasst ist, durch die durch den Wurzelteil des chirurgischen Stifts definierte Durchgangsbohrung inseriert zu werden;
wobei
durch Anwenden äußerer Kraft an dem Steckgriff, der chirurgische Stift darauf angepasst ist, in einen Knochen getrieben zu werden, um einen frakturierten Bereich zu halten; und
der chirurgische Stift, der Führungsstift und der verstärkende Metalldraht dafür geeignet sind, gekrümmt zu werden.

9. Stiftanordnung gemäß Anspruch 8, wobei der chirurgische Stift aus Edelstahl, Titan oder einer Legierung auf Titanbasis hergestellt ist.

10. Stiftanordnung gemäß Anspruch 8, wobei die Stiftanordnung so angepasst ist, dass eine Medikation oder ein Knochenzement in dem chirurgischen Hohlstift verbleiben kann, nachdem der chirurgische Stift in den Knochen inseriert ist.

11. Stiftanordnung gemäß Anspruch 8, wobei die Medikation oder der Knochenzement durch die in dem chirurgischen Stift definierten Löcher in den Knochen injiziert werden kann.

12. Stiftanordnung gemäß Anspruch 11, wobei die Medikation ausgewählt ist aus der Gruppe, bestehend aus Alkohol, Flüssigstickstoff, Antikrebs-Medikament, knochenregenerierendem Material und antiresorptivem Mittel.

## Revendications

1. Ensemble formant broche pour opération, comprenant :
une broche creuse (10) pour opération ayant une pluralité de trous latéraux (30) définis à travers une paroi de celle-ci et une partie racine (20) qui est ouverte en raison de la présence d'un trou traversant (40) ;
un élément de support creux (60) ayant une partie de fixation de broche (65) formée en son sein en forme d'une rainure pour fixer une extrémité de la broche pour opération, et étant ouvert aux deux extrémités de celui-ci ;
un percuteur en forme de T (80) conçu pour être inséré dans un trou d'insertion (63) défini à travers l'élément de support, dans lequel la broche pour opération est conçue pour être fixée à la partie de fixation de broche de l'élément de support, et en appliquant une force externe au percuteur, la broche pour opération est conçue pour être insérée dans un os ; **caractérisé en ce que** le percuteur en forme de T a un trou traversant (83) défini à travers une partie centrale de celui-ci ; et l'ensemble formant broche comprend en outre :
un pointeau de guidage (70) ou un fil métallique de renfort conçu pour être inséré à travers le trou traversant défini à travers la partie racine de la broche pour opération,
dans lequel :
le pointeau de guidage ou fil métallique de renfort est conçu pour renforcer la résistance de la broche pour opération in situ ; et
la broche pour opération, le pointeau de guidage et le fil métallique de renfort sont conçus pour être pliés.

2. Ensemble formant broche selon la revendication 1, dans lequel l'ensemble formant broche est conçu de sorte que des médicaments ou du ciment à os peuvent rester dans la broche creuse pour opération après que la broche pour opération est insérée dans l'os.

3. Ensemble formant broche selon la revendication 1 ou 2, dans lequel la partie racine de la broche pour opération est formée de manière pointue ou émoussée.

4. Ensemble formant broche selon la revendication 1 ou 2, dans lequel un filet (50) est formé sur la partie racine de la broche pour opération.

5. Ensemble formant broche selon la revendication 1 ou 2, dans lequel la broche pour opération est faite d'acier inoxydable, de titane, ou d'un alliage à base de titane.

6. Ensemble formant broche selon la revendication 1 ou 2, dans lequel les médicaments ou le ciment à os peuvent être injectés à travers les trous définis dans la broche pour opération jusque dans l'os.

7. Ensemble formant broche selon la revendication 6, dans lequel les médicaments sont sélectionnés à partir du groupe constitué par l'alcool, l'azote liquide, un médicament anticancéreux, un matériau de régénération osseuse et un agent anti-résorption.

8. Ensemble formant broche pour opération, comprenant :
une broche creuse (10) pour opération ayant une pluralité de trous latéraux (30) définis à travers une paroi de celle-ci et une partie racine (20) qui est ouverte en raison de la présence d'un trou traversant (40) ; **caractérisé en ce qu'**un filet est formé sur la partie racine, et l'ensemble formant broche pour opération comprend en outre :
un dispositif d'entraînement (90) conçu pour être ajusté dans une extrémité ouverte de la broche pour opération pour transmettre une force externe à la broche pour opération, le dispositif d'entraînement ayant un trou traversant (94) ; et
un pointeau de guidage (70) ou un fil métallique de renfort conçu pour être inséré à travers le trou traversant défini à travers la partie racine de la broche pour opération ;
dans lequel :
en appliquant une force externe au dispositif d'entraînement, la broche pour opération est conçue pour être entraînée dans un os pour maintenir une partie fracturée ; et
la broche pour opération, le pointeau de guidage et le fil métallique de renfort sont conçus pour être pliés.

9. Ensemble formant broche selon la revendication 8, dans lequel la broche pour opération est faite d'acier inoxydable, de titane, ou d'un alliage à base de titane.

10. Ensemble formant broche selon la revendication 8, dans lequel l'ensemble formant broche est conçu de sorte que les médicaments ou le ciment à os peuvent rester dans la broche creuse pour opération après que la broche pour opération est insérée dans l'os.

11. Ensemble formant broche selon la revendication 8, dans lequel des médicaments ou du ciment à os peuvent être injectés à travers les trous définis dans la broche pour opération jusque dans l'os.

12. Ensemble formant broche selon la revendication 11, dans lequel les médicaments sont sélectionnés à partir du groupe constitué par l'alcool, l'azote liquide, un médicament anticancéreux, un matériau de régénération osseuse et un agent anti-résorption.
